# EUROPÄISCHE PATENTANMELDUNG

(11) **EP 4 020 497 A1**
(43) Veröffentlichungstag der Anmeldung: **29.06.2022**
(21) Anmeldenummer: 20217460.3
(22) Anmeldetag: 28.12.2020
(51) Int. Cl.: G16H 50/80, H04W 4/029

(54) **VERFAHREN ZUR DATENVERARBEITUNG VON KONTAKTDATEN, ENDGERÄT, SERVER, NETZWERKANORDNUNG SOWIE COMPUTERPROGRAMM**

(71) Anmelder: Deutsche Telekom AG, 53113 Bonn (DE)
(72) Erfinder: Dr. WITYCH, Michael, 53227 Bonn (DE)
(74) Vertreter: Braun-Dullaeus Pannen Emmerling Patent- & Rechtsanwaltspartnerschaft mbB

(57) **Zusammenfassung**

Mit Beginn des Jahres 2020 hat sich das Bedürfnis ergeben, Kontaktinformationen zwischen Menschen zu sammeln, zu verarbeiten und zu teilen, um etwaige Kontakte ermitteln zu können, die zu einer Übertragung von Coronainfektionen geführt haben. Es ist Aufgabe der vorliegenden Erfindung eine Datenverarbeitung von Kontaktdaten weiter zu verbessern.

Hierzu wird ein Verfahren zur Datenverarbeitung von Kontaktdaten vorgeschlagen, wobei auf einem Endgerät 2 Kontaktdaten zu mindestens einem Kontaktendgerät 3 erfasst werden, wobei das Endgerät 2 und das Kontaktendgerät 3 jeweils Gerätekenndaten aufweisen, wobei die Kontaktdaten von dem Endgerät 2 zu einem Server 4 übermittelbar sind, wobei die Gerätekenndaten von dem Kontaktendgerät 2 an den Server übermittelt werden, wobei der Server 4 die Behandlung der Kontaktdaten von dem Kontaktendgerät 3 in Abhängigkeit der Gerätekenndaten des Kontaktendgeräts 3 durchführt.

## Beschreibung

Die Erfindung betrifft ein Verfahren zur Datenverarbeitung von Kontaktdaten mit den Merkmalen des Anspruchs 1. Die Erfindung betrifft des Weiteren ein Endgerät, einen Server, eine Netzwerkanordnung sowie ein entsprechendes Computerprogramm.

Mit Beginn des Jahres 2020 hat sich das Bedürfnis ergeben, Kontaktinformationen zwischen Menschen zu sammeln, zu verarbeiten und zu teilen, um etwaige Kontakte ermitteln zu können, die zu einer Übertragung von Corona geführt haben.

Die sogenannte Corona-Warn-App ist eine App für ein Smartphone, welche über eine Bluetooth Verbindung weitere Smartphones in der Umgebung sucht und über bekannte Verfahren eine Entfernung zu den weiteren Smartphones abschätzt sowie eine Dauer der Verbindung aufnimmt. Ferner wird eine ID der weiteren Smartphones diesen Daten zugeordnet. Für den Fall, dass ein Benutzer von einem Smartphone mit einer abgespeicherten ID eine Corona Erkrankung in seinem Smartphone bestätigt, wird dessen ID und ein bestimmter Zeitpunkt in seiner Corona Erkrankung, z.B. der Tag seiner ersten Symptome (DSO = Day Symptoms Onset) an alle teilnehmenden Smartphones übermittelt. In dem eigenen Smartphone wird bei einer Übereinstimmung der ID auf Basis der abgeschätzten Entfernung sowie der Dauer und dem zeitlichen Abstand einer solchen sogenannten Einzel-Dauer zum DSO ein Infektionsrisiko bestimmt und auf dem Smartphone angezeigt. Hierbei können auch mehrere Einzel-Dauern, die zeitlich unterbrochen sind, zu einem Gesamt-Infektionsrisiko einer Einzelperson - aufgrund aller zeitlich relevanten Kontakte zu einer anderen Person - in dem Smartphone verarbeitet werden. Die Übertragung des DSO und seine Berücksichtigung im empfangenen Smartphone wird hierbei nur benötigt, wenn eine reales Infektionsrisiko bestimmt werden soll. Sollen nur Nutzerverhalten bezüglich des Abstands und der Dauer innerhalb Abstandsintervalls bestimmt werden, so wird die DSO Übertragung und seine Berücksichtigung nicht benötigt.

Es ist Aufgabe der vorliegenden Erfindung eine Datenverarbeitung von Kontaktdaten weiter zu verbessern.

Diese Aufgabe wird durch ein Verfahren mit dem Merkmal des Anspruchs 1, durch ein Endgerät mit den Merkmalen des Anspruchs 12, durch einen Server mit den Merkmalen des Anspruchs 13 sowie durch eine Netzwerkanordnung mit den Merkmalen des Anspruchs 14 sowie durch ein Computerprogramm mit den Merkmalen des Anspruchs 15 gelöst.

Bevorzugte oder vorteilhafte Ausführungsformen der Erfindung ergeben sich aus den Unteransprüchen, der nachfolgenden Beschreibung sowie der beigefügten Figur.

Gegenstand der Erfindung ist somit ein Verfahren zur Datenverarbeitung von Kontaktdaten. Besonders bevorzugt dient das Verfahren zur Datenverarbeitung der Kontaktdaten zur Durchführung von Studien über das Kontaktverhalten von Menschen, z.B. in einer Großstudie. Somit geht das Verfahren davon aus, dass Kontaktdaten an eine gemeinsame Sammelstelle, in diesem Fall an mindestens einen Server, insbesondere in einer Cloud, gesammelt werden, um diese auswerten zu können.

Im Rahmen des Verfahrens werden auf einem Endgerät Kontaktdaten zu mindestens einem Kontaktendgerät erfasst. Vorzugsweise werden Kontaktdaten zu mehreren Kontaktendgeräten erfasst. Die Kontaktdaten sind den Kontaktendgeräten jeweils zugeordnet. Bei dem Endgerät und/oder dem Kontaktendgerät handelt es sich insbesondere um Smartphones, in Weiterentwicklung kann es sich auch um beliebiges User Equipment (UE) handeln. Optional ergänzend werden zu den Kontaktdaten Identifikationsdaten, insbesondere eine ID, des Kontaktendgeräts erfasst. Bei der ID kann es sich um eine statische ID handeln, vorzugsweise ist die ID jedoch als eine wechselnde ID ausgebildet. Diese IDs können auch aus Datenschutzgründen bei der Erzeugung verschlüsselt sein.

Es ist vorgesehen, dass das Endgerät und das Kontaktendgerät jeweils Gerätekenndaten aufweisen. Insbesondere umfassen die Gerätekenndaten Hardwareinformationen und/oder Softwareinformationen zu dem jeweiligen Gerät. Vorzugsweise ist das Endgerät ausgebildet, Gerätekenndaten des Kontaktendgeräts ergänzend zu den Kontaktdaten des gleichen Kontaktendgeräts zu erfassen.

Die Kontaktdaten können von dem Endgerät an einen Server übertragen werden. Ob die Kontaktdaten übertragen werden, hängt von der Datenverarbeitung der Kontaktdaten ab. Der Server und das Endgerät sowie optional ergänzend das mindestens eine Kontaktendgerät bilden eine Netzwerkanordnung.

Im Rahmen der Erfindung wird vorgeschlagen, dass die Gerätekenndaten von dem Kontaktendgerät an den Server übermittelt werden, wobei der Server die Behandlung der Kontaktdaten von dem Kontaktendgerät in Abhängigkeit der Gerätekenndaten des Kontaktendgeräts durchführt. Insbesondere bilden die Gerätekenndaten des Kontaktendgeräts eine Grundlage für die Entscheidung über die Behandlung der Kontaktdaten.

Es ist dabei eine Überlegung der Erfindung, dass die Qualität der Erfassung der Kontaktdaten stark von der Hardware und/oder der Software von dem Endgerät, aber auch von dem Kontaktendgerät abhängt. Die Qualität der Erfassung der Kontaktdaten kann beispielsweise von dem Vorhandensein einer speziellen Schnittstelle, die im Betriebssystem integriert ist, abhängen. Alternativ oder ergänzend kann die Qualität der Erfassung der Kontaktdaten z.B. von besonderen Eigenschaften des Gehäuses des Endgeräts bzw. Kontaktendgeräts oder anderen Hardwareeigenschaften abhängen, die z.B. zu einer richtungsabhängigen Erfassung der Kontaktdaten führt.

Beispielsweise können die Kontaktdaten eine Geräteentfernung zwischen dem Endgerät und dem Kontaktendgerät aufweisen, wobei die Bestimmung der Geräteentfernung in Abhängigkeit einer Dämpfung, insbesondere einer richtungsabhängigen Dämpfung variieren kann. Dabei muss beachtet werden, dass das Kontaktendgerät bzw. das Endgerät prinzipiell nicht dafür ausgelegt sind, Kontaktdaten, insbesondere Geräteentfernungen zu bestimmen, sondern Geräte spezifische Funktionen, wie zum Beispiel Internetdatenverkehr Gesprächsdatenverkehr etc. umsetzen soll. Die Erfassung von Kontaktdaten ist eine Funktion, die unter Nutzung der vorhandenen Hardware umgesetzt wurde, welche für diese Funktion jedoch nicht optimiert wurde. Somit existieren Kontaktendgeräte und/oder Endgeräte, welche diese Funktion nur ungenügend umsetzen und somit insbesondere bei groß angelegten Untersuchungen eher als Fehlerquellen statt als verwertbare Datenquellen klassifiziert werden müssen. In Kenntnis der Gerätekenndaten von dem Kontaktendgerät kann der Server entscheiden, wie mit den Kontaktdaten zu verfahren ist, insbesondere ob diese abgelehnt, verworfen oder entsprechend gekennzeichnet gespeichert werden sollen.

Durch die Übersendung der Gerätekenndaten von dem Kontaktendgerät werden somit insbesondere groß angelegte Untersuchungen über das Kontaktverhalten von Menschen, insbesondere der Besitzer von dem Endgerät und/oder den Kontaktendgeräten, qualitativ verbessert.

Bei einer bevorzugten Ausgestaltung der Erfindung erfolgt die Behandlung der Kontaktdaten des Kontaktendgeräts so, dass die Kontaktdaten in Abhängigkeit der Gerätekenndaten des Kontaktendgeräts akzeptiert oder verweigert werden. Beispielsweise kann dem Endgerät von dem Server mitgeteilt werden, dass die Kontaktdaten verweigert werden, so dass diese Kontaktdaten nicht von dem Endgerät übermittelt werden. Neben der qualitativen Verbesserung der von dem Server gesammelten Kontaktdaten, wird der Datenverkehr zwischen Endgerät und Server reduziert.

Bei einer alternativen Ausgestaltung der Erfindung ist der Server ausgebildet, die Kontaktdaten des Kontaktendgeräts in Abhängigkeit der Gerätekenndaten des Kontaktendgeräts zu verwerfen. Für den Fall, dass die Bewertung der Gerätekenndaten zeitintensiv und/oder rechenintensiv sind und/oder für den Fall, dass die Kommunikation zwischen dem Endgerät und dem Server vereinfacht werden soll, ist vorgesehen, dass der Server die Kontaktdaten von dem Kontaktendgerät zunächst entgegennimmt und nachfolgend teilweise oder vollständig verwirft.

Ferner kann vorgesehen sein, dass die Kontaktdaten des Kontaktendgeräts vom Server entgegengenommen werden und in Abhängigkeit der Gerätekenndaten des Kontaktendgeräts weiterverarbeitet, zum Beispiel korrigiert werden. So ist es denkbar, dass Verbesserungsroutinen, welche abhängig von den Gerätekenndaten des Kontaktendgeräts sind, über die Kontaktdaten prozessiert werden, um diese zu verbessern. Beispielsweise können die Verbesserungsroutinen eine Normierung der Kontaktdaten umfassen.

Bei einer besonders bevorzugten Ausgestaltung der Erfindung erfolgt die Behandlung der Kontaktdaten von dem Kontaktendgerät zusätzlich in Abhängigkeit der Gerätekenndaten des Endgeräts. Insbesondere erfolgt die Behandlung der Kontaktdaten, zum Beispiel wie diese zuvor beschrieben wurde, in Abhängigkeit der Kombination von Gerätekenndaten des Kontaktendgeräts und von Gerätekenndaten des Endgeräts. In dieser Ausgestaltung können Besonderheiten, zum Beispiel Inkompatibilitäten, zwischen dem Endgerät und dem Kontaktendgerät berücksichtigt werden. So können z.B. Probleme bei der Validität der Kontaktdaten, welche auf eine spezifische Kombination der Software, insbesondere der Betriebssysteme, im Speziellen der Version der Betriebssysteme, und/oder der Hardware entsprechend berücksichtigt werden.

Es kann dabei vorgesehen sein, dass das Endgerät die eigenen Gerätekenndaten mehrmals an den Server sendet. Alternativ kann auch vorgesehen sein, dass das Endgerät bei dem Server bekannt ist, zum Beispiel indem das Endgerät ein Konto in dem Server aufweist, so dass nur eine Identifikation (ID) von dem Endgerät übertragen werden muss, um den Server in die Lage zu versetzen, eine Kombination von Endgerät und Kontaktendgerät zu bewerten.

Es ist bevorzugt vorgesehen, dass der Server Zugriff auf ein Regelwerk, zum Beispiel eine Datenbank, hat, wobei in dem Regelwerk Vorgaben zur Behandlung der Kontaktdaten in Abhängigkeit der Gerätekenndaten des Kontaktendgeräts und/oder in Abhängigkeit der Gerätekenndaten des Endgeräts und/oder in Abhängigkeit der Kombination der Gerätekenndaten des Kontaktendgeräts und des Endgeräts vorhanden sind. Somit kann der Server in Kenntnis der jeweiligen Gerätekenndaten aus dem Regelwerk die zugeordnete Behandlung der Kontaktdaten entnehmen.

Bei einer bevorzugten Realisierung der Erfindung sind die Kontaktdaten auf Basis einer Geräteentfernung zwischen dem Endgerät und dem Kontaktendgerät und/oder einer Kontaktdauer zwischen dem Endgerät und dem Kontaktendgerät gebildet. Beispielsweise können derartige Geräteentfernungen über BLE (Bluetooth Low Energy) erfasst werden, wie dies zum Beispiel aus der Corona-Warn-App bekannt ist. Die Kontaktdaten können eine Geräteentfernung in Längeneinheiten, jedoch auch eine Signalintensität oder andere Daten umfassen, aus denen die Geräteentfernung und/oder Kontaktdauer abgeleitet werden kann.

Ferner ist es bevorzugt, dass die Gerätedaten eine Betriebssystemnummer und/oder Kennzeichnung der Betriebssystemnummer und/oder eine Gerätekennung zur Identifizierung der Hardware oder Teilen des Geräts umfassen. Somit können die Gerätedaten detaillierte Informationen über die Software und/oder die Hardware des jeweiligen Geräts umfassen.

Bei einer bevorzugten programmtechnischen Umsetzung sind die Gerätedaten insbesondere von dem Kontaktendgerät und/oder dem Endgerät in einem http-Header von einem http-Request enthalten. http-header (Kopfzeilen) erlauben es dem Endgerät zusätzliche Informationen bei einer Anfrage (http-Request) an den Server zu übergeben. Auf diese Weise ist die Kommunikation zwischen dem Endgerät und dem Server besonders einfach umzusetzen.

Bei einer möglichen Ausgestaltung der Erfindung übergibt das Endgerät die Kontaktdaten zusammen mit den zugeordneten Gerätedaten an den Server. Insbesondere werden die Kontaktdaten und die Gerätedaten in einem Paket an den Server gesendet. Der Server kann dann - wie zuvor beschrieben - das gesamte Paket weiterverarbeiten oder ablehnen.

Bei einer alternativen Ausgestaltung ist das Endgerät ausgebildet, dem Server eine Anfrageliste an Gerätedaten zu unterschiedlichen Kontaktendgeräten zu übermitteln. Der Server ist ausgebildet, dem Endgerät eine Antwortliste mit einer Auswahl der Kontaktendgeräte der Anfrageliste zu übermitteln, wobei das Endgerät ausschließlich Kontaktdaten zu den Kontaktendgeräten der Antwortliste an den Server übermittelt. Ein weiterer Gegenstand der Erfindung betrifft ein Endgerät, wobei das Endgerät programmtechnisch ausgebildet ist, Kontaktdaten und optional ergänzend Gerätekenndaten zu mindestens einem Kontaktendgerät zu erfassen und die Kontaktdaten und die Gerätekenndaten des Kontaktendgeräts an einen Server zu übermitteln.

Ein weiterer Gegenstand der Erfindung betrifft einen Server, wobei der Server programmtechnisch ausgebildet ist, Kontaktdaten und optional ergänzend Gerätekenndaten zu mindestens einem Kontaktendgerät von einem Endgerät zu empfangen.

Ein weiterer Gegenstand der Erfindung bildet eine Netzwerkanordnung, wobei die Netzwerkanordnung anordbar ist und das Endgerät, den Server und optional ergänzend das Kontaktendgerät aufweist, wobei die Netzwerkanordnung ausgebildet ist, das zuvor beschriebene Verfahren durchzuführen.

Ein weiterer Gegenstand der Erfindung wird durch ein Computerprogramm gebildet, wobei das Computerprogramm ausgebildet ist, das zuvor beschriebene Verfahren auszuführen, wenn das Computerprogramm auf der Netzwerkanordnung abläuft.

Weitere Merkmale, Vorteile und Wirkungen der Erfindung ergeben sich aus der nachfolgenden Beschreibung bevorzugter Ausführungsbeispiele der Erfindung. Dabei zeigt:

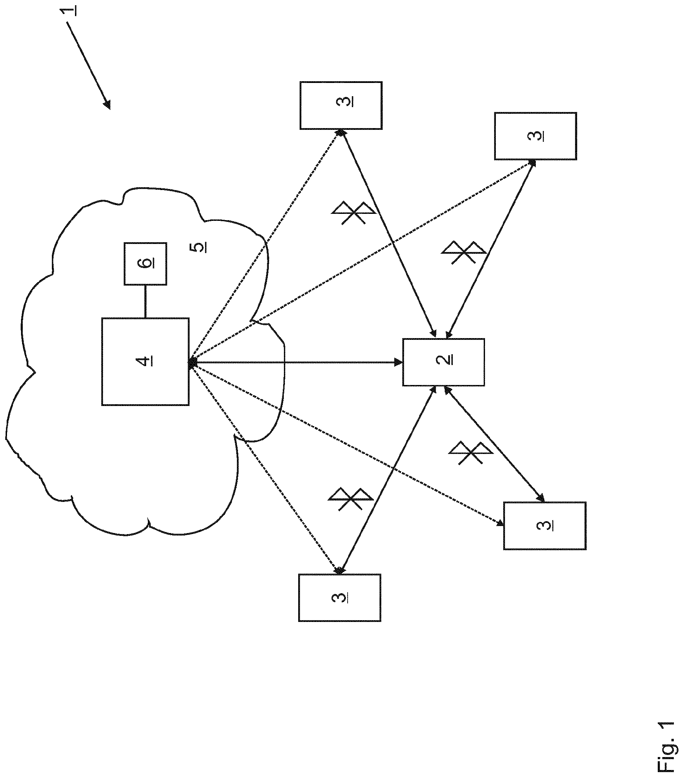

- Figur 1: eine schematische Darstellung einer Netzwerkanordnung als ein Ausführungsbeispiel der Erfindung.

Die Figur 1 zeigt in einer schematischen Darstellung eine Netzwerkanordnung 1 als ein Ausführungsbeispiel der Erfindung. Die Netzwerkanordnung 1 umfasst mindestens ein Endgerät 2, welches als ein Smartphone oder ein anderes User Equipment ausgebildet ist. Ferner weist die Netzwerkanordnung 1 eine Mehrzahl von Kontaktendgeräten 3. Alternativ hierzu kommuniziert die Netzwerkanordnung 1 mit den Kontaktendgeräten 3. Die Kontaktendgeräte 3 können ebenfalls als Smartphones oder andere User Equipment ausgebildet sein. Prinzipiell können das Endgerät 2 und die Kontaktendgeräte 3 auch baugleich realisiert sein.

Die Netzwerkanordnung 1 weist mindestens einen Server 4 auf, wobei der Server 4 beispielsweise Teil einer Cloud 5 bilden kann. Es können auch mehrere Server 4 und/oder unterschiedliche Clouds 5 in der Netzwerkanordnung 1 vorgesehen sein.

Das Endgerät 2 und optional ergänzend die Kontaktendgeräte 3 sind datentechnisch mit dem Server 4 verbunden. Die Verbindung kann kabellos oder kabelgebunden, insbesondere über Internet erfolgen. Die Verbindung ist insbesondere bidirektional ausgebildet.

Das Endgerät 2 ist ausgebildet, eine Distanzmessung zu den Kontaktendgeräten 3 durchzuführen, um eine Geräteentfernung zwischen dem Endgerät 2 und dem jeweiligen Kontaktendgerät 3 zu bestimmen. Die Distanzmessung wird beispielsweise über Bluetooth Low Energy durchgeführt. Hierbei verbindet sich das Endgerät 2 mit dem jeweiligen Kontaktendgerät 3 und ermittelt über die Empfangsamplitude die zu bestimmende Distanz. Bei der Verbindung wird zu dem eine ID sowie Gerätekenndaten wechselseitig ausgetauscht. Die Gerätekenndaten umfassen Hardwareinformationen und Softwareinformationen zu dem jeweiligen Gerät 2, 3. Ferner nimmt das Endgerät 2 die Kontaktdauer zwischen dem Endgerät 2 und dem jeweiligen Kontaktendgerät 3 auf. Die Kontaktendgeräte 3 führen das gleiche Verfahren durch, so dass diese in Bezug auf das Endgerät 2 die entsprechenden Daten vorliegen haben. Ferner führen die Kontaktendgeräte 3 das gleiche Verfahren untereinander durch. Im Ergebnis liegt in den Geräten 2, 3 jeweils eine Geräteentfernung, eine Kontaktdauer sowie eine Identifikationsmöglichkeit, insbesondere ID, des kontaktierten Geräts 2, 3 vor.

Allerdings sind nicht alle Geräte 2, 3 und insbesondere nicht alle Kombinationen zwischen dem Endgerät 2 und einem der Kontaktendgeräte 3 für eine verlässliche Distanzmessung geeignet. Die fehlende Kompatibilität zwischen den Geräten 2, 3 kann zum einen in der hardwaretechnischen Ausstattung, wie zum Beispiel eine ungünstige Integration der Schnittstelle, insbesondere der Bluetooth-Schnittstelle, in dem jeweiligen Gerät 2,3 sein. Ferner kann die fehlende Kompatibilität durch die softwaretechnische Ausstattung, z.B. unterschiedliche oder unverträgliche Betriebssystemvarianten bzw. -versionen entstehen. Die Gerätekenndaten enthalten entsprechende Hardwareinformationen und/oder Softwareinformationen. Ein Beispiel für die Information in den Gerätekenndaten ist das Vorhandensein einer speziellen Schnittstelle, die im Betriebssystem integriert ist, und/oder die besonderen Eigenschaften der Einbettung ins Gehäuse des Geräts 2, 3 und die dadurch verursachte hohe Dämpfung und/oder unterschiedliche Dämpfung in unterschiedliche Richtungen.

Die Kontaktdaten sollen bestimmungsgemäß zum Server 4 übertragen werden, um dort in der Gesamtheit ausgewertet werden zu können. Allerdings ergibt sich bei der Betrachtung der fehlenden Kompatibilität zwischen den Geräten 2, 3 der Bedarf, Kontaktdaten von inkompatiblen Geräten 2, 3 möglichst effektiv auszufiltern.

Nachdem die Kenntnis über die inkompatiblen Geräte 2, 3 nicht auf den Geräten 2, 3 selbst vorhanden ist, muss für eine derartige Filterung der Server 4 Unterstützung leisten. Der Server 4 weist beispielsweise ein Regelwerk 6 auf oder ist mit diesem verbunden, wobei in dem Regelwerk 6 die Geräte 2, 3 und insbesondere Kombinationen von Geräten 2, 3 erfassbar sind und Daten zur Behandlung der Kontaktdaten in Abhängigkeit der Geräte 2, 3 bzw. der Kombinationen von Geräten 2, 3.

Das Endgerät 2 und optional auch die Kontaktendgeräte 3 sind ausgebildet, die Gerätekenndaten oder Teile davon an den Server 4 zusenden. Dieser ist ausgebildet, die Gerätekenndaten von dem Kontaktendgerät 3 und von dem Endgerät 2 als eine Gerätekombination in dem Regelwerk 6 zu prüfen und Daten zur Behandlung der Kontaktdaten zu erhalten.

Die Behandlung der Kontaktdaten kann zum einen so erfolgen, dass der Server 4 dem Endgerät 2 mitteilt, dass die Kontaktdaten gar nicht übermittelt werden sollen. In diesem Fall können Übertragungskapazitäten zwischen dem Endgerät 2 und dem Server 4 eingespart werden.

Zum anderen ist es möglich, dass die Kontaktdaten gemeinsam mit den Gerätekenndaten an den Server 4 übermittelt werden und der Server 4 die entsprechenden Kontaktdaten verwirft.

Beispielsweise können die Kontaktdaten gemeinsam mit den Gerätekenndaten als Paket versendet werden. Hierbei ist es beispielsweise möglich, dass die Gerätekenndaten in einem http-Header angeordnet sind, so dass das Paket nach Prüfung des http-Headers ggf. schnell verworfen werden kann.

Für den Fall, dass die Gerätekenndaten, insbesondere die Gerätekenndaten der Gerätekombination, gemäß Regelwerk 6 zu validen Kontaktdaten führen, werden diese in dem Server 4 weiterverarbeitet oder zur Verarbeitung weitergeleitet.

In dem Ausführungsbeispiel wird der Server 4, der die Kontaktdaten von entsprechenden Apps auf den Geräten 2, 3 aggregiert, nur die Datensätze der Apps bzw. Geräte 2, 3 annehmen, wenn im verlangten Anfrage http-Request im http-header der Anfrage der App die ausreichend hohe Betriebssystemnummer der akzeptierten Betriebssysteme enthalten ist, so dass eine entsprechende Schnittstelle in dem Betriebssystem integriert ist und in der verlangten Anfrage beim http-Request im http-header der Anfrage der App eine Gerätekennung enthalten ist, die der Server akzeptiert. Denn der Server 4 hat z.B. eine Tabelle als Regelwerk 6 vorliegen an Hand der er die Mindestqualität einer elektromagnetischen Distanzmessung zwischen zwei Geräten 2, 3 prüfen kann. Da immer zwei Geräte 2, 3 bei der Distanzmessung involviert sind, muss die App von dem Endgerät 2 (A), wenn sie deren Kontaktdaten, die auf AppA-zu-AppX Distanzmessungen mit X Elementen aus {B,C,D...} als weitere Kontaktendgeräte 3 beruhen, an den Server 4 senden will, in ihren http-Anfrage Header von beiden Geräten 2, 3 (sich selbst als Endgerät 2, also A) und von der jeweiligen Gegenstelle X als Kontaktendgerät 3 die Informationen zum Betriebssystem und zum Device Namen als Gerätekenndaten angeben. Die App wird dann bei der Kontaktherstellung und http Pakete Übermittlung entweder:
- ihre Daten nacheinander in http-Paketen senden, die jeweils nur eine feste A-zu-X Kombination enthalten, so dass der Server 4 auch Pakete ablehnen kann oder dem Server 4 vorab eine Liste der A + {B,C,D...} Geräten 2, 3 mit ihren jeweils zwei Parametern OS u. Devicename als Gerätekenndaten senden, worauf der Server 4 dann mitteilen kann, welche der Gerätekombinationen er akzeptieren wird. Die App auf dem Endgerät 2 (A) kann dann vorab ihre Daten ausdünnen, damit sie keines ihrer zukünftigen http Pakete abgelehnt bekommt. Die Annahme oder Ablehnung durch den Server 4 bestimmt sich insbesondere durch die absoluten und relativen Dämpfungseigenschaften der Geräte 2, 3 bei aktuell eingestellter Sendestärke und auch die Unterschiede dieser Werte in Abhängigkeit der Ausstrahlungsrichtung. Und der Server 4 kann hierzu Schwellwerte definieren, die er aber auch in Abhängigkeit der Genauigkeit einer aktuellen Großstudie treffen wird, das heißt es kann vorgesehen sein, dass die Großstudie aus der Anfrage der App A von dem Endgerät 2 bei dem Server 4 auch hervorgeht. Gleiche Dämpfungseigenschaften in alle Raumrichtungen sind besser als unterschiedliche, da bei der Device-zu-Device Messung z. B. mittels BLE die Raumrichtung zwischen den zwei Geräten relativ zu einem Device-internen, Device-festen Koordinatensystem nicht bekannt ist. Diese Eigenschaften gehen aus dem technischen Datenblatt eines Gerätes 2 hervor. Der Server 4 wird hierbei die Kombination von A mit einem X bewerten und nur die Kombinationen akzeptieren, die besser als ein Schwellwert sind. Je kürzer, die zu messenden Strecken sind desto eher wirken sich diese Geräteunterschiede aus, da es unterschiedliche Additionskonstanten bzgl. der Ausgangs, sendestärke sind, wobei der der Zusammenhang zwischen Ausgangssendestärke zu empfangener gedämpfter Stärke bzgl. der Entfernung nicht linear. Bei Messungen von 0-1,5m Abstand wie bei Viren-Apps ist es mindestens für den Ausschluss älterer Geräte wichtig oder für den Ausschluss von Geräten deren technisches Datenblatt unzureichend detaillierte Angaben aufweist.

Die Kontaktdaten können von dem Server 4 zum Beispiel für eine Großstudie zu dem Kontaktverhalten von Menschen eingesetzt werden. Dabei ist es auch möglich, dass das Endgerät 2 und/oder das Kontaktendgerät 3 bereits vorverarbeitete und/oder aggregierte Datensätze sendet. Beispielsweise können alle Kontaktdaten von dem Endgerät 2 zu einem der Kontaktendgeräte 3 von einem vorgebbaren Zeitraum gesammelt und als ein gemeinsamer Datensatz als die Kontaktdaten übertragen werden oder zumindest zur Übertragung angeboten werden.

### Bezugszeichenliste

- 1: Netzwerkanordnung
- 2: Endgerät
- 3: Kontaktendgerät
- 4: Server
- 5: Cloud
- 6: Regelwerk

## Patentansprüche

1. Verfahren zur Datenverarbeitung von Kontaktdaten,
wobei auf einem Endgerät (2) Kontaktdaten zu mindestens einem Kontaktendgerät (3) erfasst werden,
wobei das Endgerät (2) und das Kontaktendgerät (3) jeweils Gerätekenndaten aufweisen,
wobei die Kontaktdaten von dem Endgerät (2) zu einem Server (4) übermittelbar sind, **dadurch gekennzeichnet, dass**
die Gerätekenndaten des Kontaktendgeräts (3) von dem Endgerät (2) an den Server (4) übermittelt werden, wobei der Server (4) die Behandlung der Kontaktdaten von dem Kontaktendgerät (3) in Abhängigkeit der Gerätekenndaten des Kontaktendgeräts (3) durchführt.

2. Verfahren nach Anspruch 1, **dadurch gekennzeichnet, dass** der Server (4) die Übermittlung der Kontaktdaten des Kontaktendgeräts (3) in Abhängigkeit der Gerätekenndaten des Kontaktendgeräts (3) verweigert.

3. Verfahren nach Anspruch 1 oder 2, **dadurch gekennzeichnet, dass** der Server (4) die Kontaktdaten des Kontaktendgeräts (3) in Abhängigkeit der Gerätekenndaten des Kontaktendgeräts (3) verwirft.

4. Verfahren nach einem der vorhergehenden Ansprüche, **dadurch gekennzeichnet, dass** die Behandlung der Kontaktdaten von dem Kontaktendgerät (3) in Abhängigkeit der Gerätekenndaten des Endgeräts (2) erfolgt.

5. Verfahren nach einem der vorhergehenden Ansprüche, **dadurch gekennzeichnet, dass** der Server (4) Zugriff auf ein Regelwerk (6) hat, wobei in dem Regelwerk (6) Daten zur Behandlung der Kontaktdaten in Abhängigkeit der Gerätekenndaten des Endgeräts (2) und/oder des Kontaktendgeräts (3) vorhanden sind.

6. Verfahren nach einem der vorhergehenden Ansprüche, **dadurch gekennzeichnet, dass** die Kontaktdaten eine Geräteentfernung zwischen dem Endgerät (2) und dem Kontaktendgerät (3) und/oder eine Kontaktdauer zwischen dem Endgerät (2) und dem Kontaktendgerät (3) umfasst.

7. Verfahren nach einem der vorhergehenden Ansprüche, **dadurch gekennzeichnet, dass** die Kontaktdaten auf Basis einer Geräteentfernung zwischen dem Endgerät (2) und dem Kontaktendgerät (3) und/oder einer Kontaktdauer zwischen dem Endgerät (2) und dem Kontaktendgerät (3) gebildet sind.

8. Verfahren nach einem der vorhergehenden Ansprüche, **dadurch gekennzeichnet, dass** die Gerätekenndaten eine Betriebssystemnummer und/oder Kennzeichnung der Betriebssystemnummer und/oder eine Gerätekennung zur Identifizierung der Hardware oder Teilen des Geräts (2, 3) umfassen.

9. Verfahren nach einem der vorhergehenden Ansprüche, **dadurch gekennzeichnet, dass** die Gerätekenndaten in einem http-Header von einem http-Request enthalten sind, welcher an den Server (4) gesendet wird.

10. Verfahren nach einem der vorhergehenden Ansprüche, **dadurch gekennzeichnet, dass** das Endgerät (2) die Kontaktdaten zusammen mit den Gerätekenndaten sendet, wobei der Server (4) ausgebildet ist, die Daten in Abhängigkeit der Gerätekenndaten abzulehnen und/oder zu verwerfen.

11. Verfahren nach einem der vorhergehenden Ansprüche 1 bis 9, **dadurch gekennzeichnet, dass** das Endgerät (2) ausbildet ist, dem Server (4) eine Anfrageliste an Gerätekenndaten zu unterschiedlichen Kontaktendgeräten (3) zu übermitteln, wobei der Server (4) ausgebildet ist, dem Endgerät (2) eine Antwortliste mit einer Auswahl der Kontaktendgeräte () zu übermitteln, so dass das Endgerät Kontaktdaten zu der Auswahl der Kontaktendgeräte übermitteln kann.

12. Endgerät (2), wobei das Endgerät (2) programmtechnisch ausgebildet ist, Kontaktdaten und optional ergänzend Gerätekenndaten zu mindestens einem Kontaktendgerät (3) zu erfassen und die Kontaktdaten und die Gerätekenndaten des Kontaktendgeräts (3) an einen Server (4) zu übermitteln.

13. Server (4), wobei der Server (4) programmtechnisch ausgebildet ist, Kontaktdaten und optional ergänzend Gerätekenndaten zu mindestens einem Kontaktendgerät (3) von einem Endgerät (2) zu empfangen.

14. Netzwerkanordnung (1) mit dem Endgerät (2) nach Anspruch 12 und dem Server (4) nach Anspruch 13, **dadurch gekennzeichnet, dass** die Netzwerkanordnung (1) zur Ausführung des Verfahrens nach einem der Ansprüche 1 zur Ausführung des Verfahrens

15. Computerprogramm, wobei das Computerprogramm ausgebildet ist, das Verfahren gemäß der Ansprüche 1 bis 12 auszuführen, wenn das Computerprogramm auf der Netzwerkanordnung (1) abläuft.

## Geänderte Patentansprüche

### Geänderte Patentansprüche gemäss Regel 137(2) EPÜ.

1. Verfahren zur Datenverarbeitung von Kontaktdaten,
wobei auf einem Endgerät (2) Kontaktdaten zu mindestens einem Kontaktendgerät (3) erfasst werden,
wobei das Endgerät (2) und das Kontaktendgerät (3) jeweils Gerätekenndaten aufweisen,
wobei die Kontaktdaten von dem Endgerät (2) zu einem Server (4) übermittelbar sind,
**dadurch gekennzeichnet, dass**
die Gerätekenndaten des Kontaktendgeräts (3) von dem Endgerät (2) an den Server (4) übermittelt werden, wobei der Server (4) die Behandlung der Kontaktdaten von dem Kontaktendgerät (3) in Abhängigkeit der Gerätekenndaten des Kontaktendgeräts (3) durchführt.

2. Verfahren nach Anspruch 1, **dadurch gekennzeichnet, dass** der Server (4) die Übermittlung der Kontaktdaten des Kontaktendgeräts (3) in Abhängigkeit der Gerätekenndaten des Kontaktendgeräts (3) verweigert.

3. Verfahren nach Anspruch 1 oder 2, **dadurch gekennzeichnet, dass** der Server (4) die Kontaktdaten des Kontaktendgeräts (3) in Abhängigkeit der Gerätekenndaten des Kontaktendgeräts (3) verwirft.

4. Verfahren nach einem der vorhergehenden Ansprüche, **dadurch gekennzeichnet, dass** die Behandlung der Kontaktdaten von dem Kontaktendgerät (3) in Abhängigkeit der Gerätekenndaten des Endgeräts (2) erfolgt.

5. Verfahren nach einem der vorhergehenden Ansprüche, **dadurch gekennzeichnet, dass** der Server (4) Zugriff auf ein Regelwerk (6) hat, wobei in dem Regelwerk (6) Daten zur Behandlung der Kontaktdaten in Abhängigkeit der Gerätekenndaten des Endgeräts (2) und/oder des Kontaktendgeräts (3) vorhanden sind.

6. Verfahren nach einem der vorhergehenden Ansprüche, **dadurch gekennzeichnet, dass** die Kontaktdaten eine Geräteentfernung zwischen dem Endgerät (2) und dem Kontaktendgerät (3) und/oder eine Kontaktdauer zwischen dem Endgerät (2) und dem Kontaktendgerät (3) umfasst.

7. Verfahren nach einem der vorhergehenden Ansprüche, **dadurch gekennzeichnet, dass** die Kontaktdaten auf Basis einer Geräteentfernung zwischen dem Endgerät (2) und dem Kontaktendgerät (3) und/oder einer Kontaktdauer zwischen dem Endgerät (2) und dem Kontaktendgerät (3) gebildet sind.

8. Verfahren nach einem der vorhergehenden Ansprüche, **dadurch gekennzeichnet, dass** die Gerätekenndaten eine Betriebssystemnummer und/oder Kennzeichnung der Betriebssystemnummer und/oder eine Gerätekennung zur Identifizierung der Hardware oder Teilen des Geräts (2, 3) umfassen.

9. Verfahren nach einem der vorhergehenden Ansprüche, **dadurch gekennzeichnet, dass** die Gerätekenndaten in einem http-Header von einem http-Request enthalten sind, welcher an den Server (4) gesendet wird.

10. Verfahren nach einem der vorhergehenden Ansprüche, **dadurch gekennzeichnet, dass** das Endgerät (2) die Kontaktdaten zusammen mit den Gerätekenndaten sendet, wobei der Server (4) ausgebildet ist, die Daten in Abhängigkeit der Gerätekenndaten abzulehnen und/oder zu verwerfen.

11. Verfahren nach einem der vorhergehenden Ansprüche 1 bis 9, **dadurch gekennzeichnet, dass** das Endgerät (2) ausbildet ist, dem Server (4) eine Anfrageliste an Gerätekenndaten zu unterschiedlichen Kontaktendgeräten (3) zu übermitteln, wobei der Server (4) ausgebildet ist, dem Endgerät (2) eine Antwortliste mit einer Auswahl der Kontaktendgeräte () zu übermitteln, so dass das Endgerät Kontaktdaten zu der Auswahl der Kontaktendgeräte übermitteln kann.

12. Endgerät (2), wobei das Endgerät (2) programmtechnisch ausgebildet ist, Kontaktdaten und ergänzend Gerätekenndaten zu mindestens einem Kontaktendgerät (3) zu erfassen und die Kontaktdaten und die Gerätekenndaten des Kontaktendgeräts (3) an einen Server (4) zu übermitteln.

13. Server (4), wobei der Server (4) programmtechnisch ausgebildet ist, Kontaktdaten und ergänzend Gerätekenndaten zu mindestens einem Kontaktendgerät (3) von einem Endgerät (2) zu empfangen.

14. Netzwerkanordnung (1) mit dem Endgerät (2) nach Anspruch 12 und dem Server (4) nach Anspruch 13, **dadurch gekennzeichnet, dass** die Netzwerkanordnung (1) zur Ausführung des Verfahrens nach einem der Ansprüche 1 bis 11 zur Ausführung des Verfahrens

15. Computerprogramm, wobei das Computerprogramm ausgebildet ist, das Verfahren gemäß der Ansprüche 1 bis 12 auszuführen, wenn das Computerprogramm auf der Netzwerkanordnung (1) abläuft.
